# EUROPEAN PATENT APPLICATION

(11) **EP 4 473 963 A1**
(43) Date of publication of application: **11.12.2024**
(21) Application number: 23749727.6
(22) Date of filing: 31.01.2023
(51) Int. Cl.: A61K 31/198, A23K 20/142, A23K 50/40, A23L 33/10, A61K 35/12, A61K 35/60, A61K 47/02, A61K 47/26, A61P 3/02, A61P 13/12, A61P 43/00

(54) **PHARMACEUTICAL OR FOOD COMPOSITION FOR ANIMALS**

(30) Priority: 01.02.2022 JP 2022013891
(71) Applicant: Inaba Shokuhin Co., Ltd., Shizuoka-shi, Shizuoka 421-3104 (JP); Fancl Corporation, Kanagawa 231-8528 (JP)
(72) Inventor: INABA, Atsuhiro, Shizuoka-shi, Shizuoka 421-3104 (JP); SHIBATA, Keiji, Shizuoka-shi, Shizuoka 421-3104 (JP); SUNAGA, Tasuku, Shizuoka-shi, Shizuoka 421-3104 (JP); SHIRAO, Daiki, Shizuoka-shi, Shizuoka 421-3104 (JP); KURAUCHI, Narumi, Shizuoka-shi, Shizuoka 421-3104 (JP); MIYAZAKI, Toru, Tokyo 162-8666 (JP)
(74) Representative: Weickmann & Weickmann PartmbB
(86) International application number: PCT/JP2023/002989
(87) International publication number: WO 2023/149411

(57) **Abstract**

The present invention provides a pharmaceutical or food composition for animals, containing a first composition and a second composition in combination, wherein the first composition contains a fish meat or livestock meat component but does not contain cystine, the second composition contains cystine but does not contain reducing sugar or phosphorus, and the first composition and the second composition are filled in separate containers.

## Description

### [Technical Field]

The present invention relates to a pharmaceutical or food composition for animals.

### [Background Art]

In recent years, attention has been focused on drug discovery based on apoptosis inhibitor of macrophage (AIM, also called "CD5L" etc.).

AIM (also called CD5L) is a protein that is specifically produced by macrophages and identified by one of the present inventors as a factor that suppresses apoptosis of the macrophage itself (Non Patent Literature 1). AIM has been suggested to be related to various diseases. For example, the blood concentration of AIM increases with obesity, and AIM is taken up into adipocytes by endocytosis via CD36, inducing the decomposition of accumulated neutral fat, suggesting a relationship with anti-obesity (Non Patent Literature 2). AIM also has the effect of releasing free fatty acids from adipocytes by decomposing neutral fats. Here, since it is known that free fatty acids stimulate toll-like receptors, causing chronic inflammation in adipose tissue and resulting in the acquisition of insulin resistance, and that the acquisition of insulin resistance is the basis for the onset of metabolic syndrome, AIM is also thought to be related to metabolic syndrome (Non Patent Literature 3).

Furthermore, the applicability of AIM to liver disease based on the mechanism by which obese AIM knockout (KO) mice loaded with a high-calorie diet exhibit pathological conditions similar to those of human NASH, including obesity, fatty liver, fibrosis of liver parenchyma, and carcinogenesis (Patent Literature 1), and the administration of AIM to AIM KO mice in which acute renal failure was caused by transient renal ischemia-reperfusion was observed to rapidly improve renal function, and the applicability of AIM to acute renal failure and chronic renal disease has been reported (Patent Literature 2) .

Furthermore, when recombinant AIM protein is administered into the brain of a disease model mouse of Alzheimer's disease, the AIM protein accumulates in amyloid plaques, and the amyloid plaques are significantly reduced by microglia (macrophages in the brain), so the applicability of AIM to neurodegenerative diseases has also been reported (Patent Literature 3).

As mentioned above, it has been suggested that AIM may be involved in various diseases, so there are high expectations for drug development using AIM, but there are also problems associated with AIM drug development. For example, as a means of delivering AIM protein to a target site in a subject, a means of administering AIM protein to the target site (protein preparation) and a means of administering a viral vector into which a nucleic acid encoding the AIM gene has been inserted (nucleic acid medicine) are expected to be used. Biopharmaceuticals such as protein preparations and nucleic acid medicines generally have a more complex production step than low-molecular-weight medicines, resulting in higher costs, and proteins and viral vectors are more unstable than small molecules, which also poses problems in terms of stability.

In consideration of these problems in AIM drug discovery, the development of pharmaceuticals that use as active ingredients substances that can enhance the activity of AIM present in the body, rather than AIM itself, is being carried out in parallel.

It has been reported that in mammals, AIM present in the blood forms a complex with IgM pentamer and is rarely present in a free form (Non Patent Literature 4). AIM bound to IgM pentamer is in an inactive state, and becomes active when released from IgM pentamer. Therefore, substances that can efficiently release AIM from IgM pentamer in the body may be promising as medicines and health foods. Compounds with thiol groups in their chemical structure have been reported as such substances (Patent Literature 4).

Incidentally, AIM is conserved in many mammals, but it is known that it has undergone unique evolution, especially in felines. Feline AIM has a much stronger binding force to IgM pentamer than human or mouse AIM, and is less likely to produce free AIM, which is an active form. Therefore, it is known that feline AIM has a very low efficiency of suppressing renal function decline, and renal failure occurs frequently (Non Patent Literature 5).

### [Citation List]

### [Patent Literature]

[Patent Literature 1]
   WO 2013/162021
[Patent Literature 2]
   WO 2015/119253
[Patent Literature 3]
   WO 2020/071318
[Patent Literature 4]
   JP-B-6864301

### [Non Patent Literature]

[Non Patent Literature 1]
   Miyazaki, J Exp Med 189:413-422, 1999
[Non Patent Literature 2]
   Kurokawa, Cell Metab 11:479-492, 2010
[Non Patent Literature 3]
   Kurokawa, PNAS 108:12072-12077, 2011
[Non Patent Literature 4]
   Miyazaki et al., Cell Reports 3, 1187-1198, 2013
[Non Patent Literature 5]
   Sugisawa et al., Sci. Rep. 6:35251, 2016

### [Summary of Invention]

### [Technical Problem]

From the above, the present invention aims to provide a drug or food at a low cost that can efficiently release AIM from IgM pentamer in the body of an animal.

### [Solution to Problem]

In the process of investigating the above-mentioned problem, the present inventors referred to the teachings of Patent Literature 4 and attempted to add cystine, a compound having a thiol group in its chemical structure, to a food composition. The present inventors produced a general animal food product using a normal process except for adding cystine, and found that hydrogen sulfide was generated in the product, making the food toxic to animals. Cystine has been proven safe as a food additive and is known to be very stable, and thus the generation of hydrogen sulfide was unexpected. Therefore, the present inventors conducted intensively studied the cause of hydrogen sulfide generation, and as a result, discovered that hydrogen sulfide was generated as a result of a combination of various conditions in the production step of animal food. The present inventors have found that, among the multiple factors that cause hydrogen sulfide to be generated, the coexistence of specific substances with cystine or the pH of an environment in which cystine is present is a particular factor that causes hydrogen sulfide to be generated. The present inventors have conducted further studies based on this finding and completed the present invention. That is, the present invention is as follows:

[1] A pharmaceutical or food composition for animals, comprising a first composition and a second composition in combination,
   wherein the first composition comprises a fish meat or livestock meat component but does not comprise cystine,
   the second composition comprises cystine but does not comprise reducing sugar or phosphorus, and
   the first composition and the second composition are filled in separate containers.
[2] The composition of [1], wherein the first composition further comprises either or both of reducing sugar and phosphorus.
[3] The composition of [1] or [2], wherein the second composition has a pH of 3 to 9.
[4] The composition of any of [1] to [3], wherein the first composition and the second composition are sterilized at a high temperature.
[5] The composition of [4], wherein the high-temperature sterilization is performed under conditions of 100 to 150°C and 1 to 180 minutes.
[6] The composition of any of [1] to [5], wherein the animal is Canidae or Felidae.
[7] The composition of [6], wherein Felidae is Felis catus.
[8] The composition of any of [1] to [7], wherein a container filled with the first composition and a container filled with the second composition are connected.
[9] A method for producing a pharmaceutical or food composition for animals, comprising a first composition and a second composition in combination, the method comprising
   a step of filling a first container with a first composition comprising a fish meat or livestock meat component but not comprising cystine, and
   a step of filling a second container with a second composition comprising cystine but not comprising reducing sugar or phosphorus.
[10] The production method of [9], wherein the first composition further comprises either or both of reducing sugar and phosphorus.
[11] The production method of [9] or [10], wherein the second composition has a pH of 3 to 9.
[12] The production method of any of [9] to [11], further comprising a step of subjecting the first container filled with the first composition and the second container filled with the second composition to high-temperature sterilization.
[13] The production method of [12], wherein the high-temperature sterilization is performed under conditions of 100 to 150°C and 1 to 180 minutes.
[14] The production method of any of [9] to [13], wherein the animal is Canidae or Felidae.
[15] The production method of [14], wherein Felidae is Felis catus.
[16] The production method of any of [9] to [15], wherein the container filled with the first composition and the container filled with the second composition are connected.

### [Advantageous Effects of Invention]

According to the present invention, a safe pharmaceutical or food composition for animals that contains cystine and does not generate hydrogen sulfide can be produced at a low cost.

### [Brief Description of Drawings]

[Fig. 1]

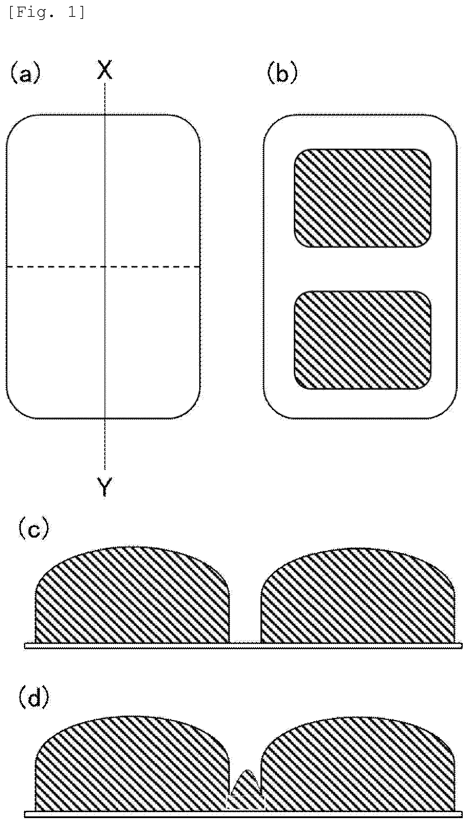

   Fig. 1 shows an example of an integrated container used in the present invention. (a) shows a schematic diagram of the container as viewed from above. The dotted line shows the part where the container can be folded. (b) shows a schematic diagram of the container as viewed from below. The part filled with the first composition or the second composition is shown by hatching. (c) shows a schematic diagram of a container in which the part filled with the first composition and the part filled with the second composition are completely separated. (d) shows a schematic diagram of a container in which a part of the part filled with the first composition and a part of the part filled with the second composition are openly connected. (c) and (d) are X-Y cross-sectional views of (a), and the part filled with the first composition or the second composition is shown by hatching.
[Fig. 2]

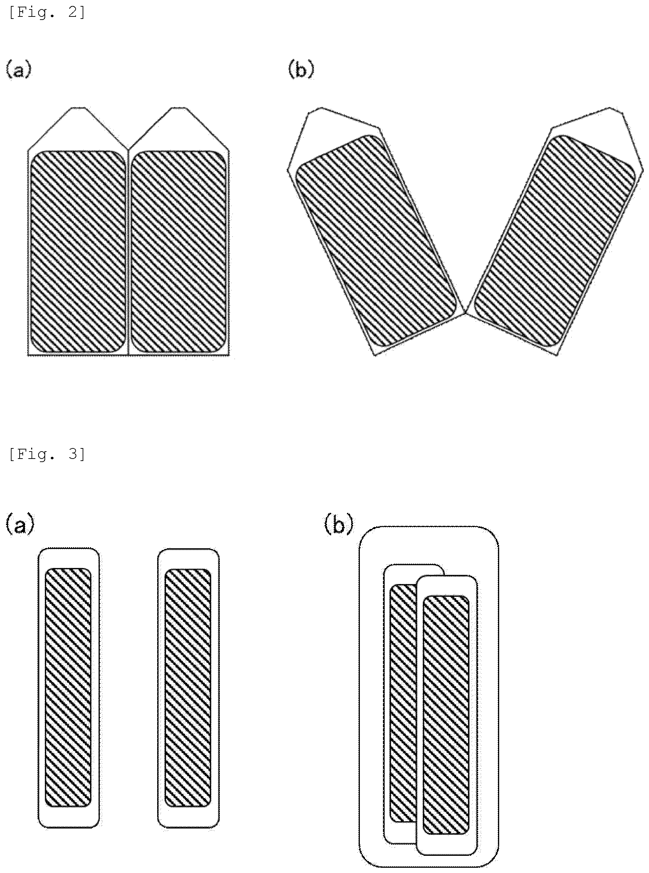

   Fig. 2 shows another embodiment of an integrated container used in the present invention. The portions filled with the first composition and the second composition are indicated by hatching. (a) shows an embodiment in which the container portion filled with the first composition and the container portion filled with the second composition are connected at one end. The connecting portion can be configured to be separable, and (b) shows an embodiment in which the container portion filled with the first composition and the container portion filled with the second composition are partially separated.
[Fig. 3]
   Fig. 3 shows one embodiment of a container used in the present invention (an embodiment in which the container filled with the first composition and the container filled with the second composition are completely separated). The portions filled with the first composition and the second composition are indicated by hatching. During the production step, the container filled with the first composition and the container filled with the second composition may be produced separately as shown in (a), and finally these may be combined and packaged in a packaging bag to prepare the pharmaceutical or food composition of the present invention.

### [Description of Embodiments]

The present invention is described in detail below. Unless otherwise specified, "%" means "% by weight".

### 1. Pharmaceutical or food composition for animals

The present invention provides a pharmaceutical or food composition for animals that is a combination of a first composition and a second composition, the first composition containing a fish meat or livestock meat component but not containing cystine, and the second composition containing cystine but not containing reducing sugars and phosphorus, characterized in that the first composition and the second composition are filled in separate containers (hereinafter sometimes referred to as the "pharmaceutical or food composition of the present invention").

The pharmaceutical or food composition of the present invention is composed of a combination of two types of compositions, the first composition and the second composition. The first composition and the second composition are filled in independent containers before being given to animals (for example, at the time of production, transportation, storage, or sale, etc.), and are maintained in an unmixed state. When an animal is to eat the pharmaceutical or food composition of the present invention, the composition is put from the independent container into another container, where the two compositions are combined and then fed to the animal. When feeding the animal, the two compositions may be mixed until they are homogeneous, or may be brought together without mixing.

### (First composition)

The fish meat or livestock meat component contained in the first composition is not particularly limited as long as it is of a grade that can be used in the production of food for animals. Examples of fish meat components include, but are not limited to, fish meat components derived from fish such as Cervidae (e.g., Tuna, Bonito), Salmonidae (e.g., chum salmon, salmon etc.), Codidae (e.g., codfish), Herringidae (e.g., Japanese sardine) and family of anchovies (e.g., Japanese anchovy) and the like. The fish meat components also include components derived from shellfish (e.g., scallop) and crustacean (e.g., crab). Examples of livestock meat components include, but are not limited to, meat components derived from livestock such as Phasianidae (e.g., chicken), Bovidae (e.g., bovine), Suidae (e.g., swine), Cervidae (e.g., deer) and Equidae (e.g., horse) and the like. In one embodiment, the first composition may contain both fish meat component and livestock meat component.

The lower limit of the amount of fish meat or livestock meat component in the first composition is generally 15% or more, preferably 20% or more or 25% or more, but is not limited to these, based on the first composition. The upper limit is generally 70% or less, preferably 60% or less or 55% or less, but is not limited to these. In one embodiment, the amount of fish meat or livestock meat component contained in the first composition is generally 15 to 70%, preferably 20 to 60% or 25 to 55%, but is not limited to these. The amount of fish meat or livestock meat component shown in the present specification is not a dry weight, but a weight including moisture.

In one embodiment, the first composition may further contain at least one selected from the group consisting of reducing sugar, phosphorus, and a thickener.

In the present specification, the "reducing sugar" refers to a sugar that forms an aldehyde group or a ketone group in a basic solution. Examples of reducing sugar include monosaccharides such as glucose, fructose, arabinose, and glyceraldehyde, and maltose-type disaccharides such as lactose and maltose, or oligosaccharides. In one embodiment of the present invention, the reducing sugar may be glucose or oligosaccharide.

When a reducing sugar is contained in the first composition, the lower limit of the amount of the reducing sugar is generally 0.01% or more, preferably 0.1% or more, 1% or more, or 3% or more, but is not limited to these. The upper limit is generally 8% or less, preferably 6% or less, 5% or less, or 4% or less, but is not limited to these. In one embodiment, the amount of reducing sugar contained in the first composition is generally 0.01 to 8%, preferably 0.1 to 6%, 1 to 5%, or 3 to 4%, but is not limited thereto.

In addition, in the present specification, "phosphorus" is a concept that includes both organic phosphorus (phosphorus contained in food materials) and inorganic phosphorus (phosphorus used as a food additive). As described above, the first composition contains a fish meat or livestock meat component, and thus it basically contains organic phosphorus, but the amount of phosphorus may be increased by adding foodstuff (e.g., eggs, milk, beans, etc.) other than the fish meat or livestock meat component to the first composition, or the amount of phosphorus may be increased by adding inorganic phosphorus as a food additive. In the present invention, when phosphorus is added, commercially available "potassium dihydrogen phosphate" (produced by Yoneyama Chemical Industry Co., Ltd.) and the like can be preferably used as inorganic phosphorus, but is not limited thereto.

When phosphorus is contained in the first composition, there is no limitation on the amount of phosphorus contained therein, and it may be set appropriately according to the property of the intended product. In addition, components such as reducing sugars and phosphorus are generally included in "seasonings" in the production of pharmaceutical products or foods for animals.

In addition, in one embodiment of the present invention, the first composition may contain a thickener. The thickener that can be used in the present invention is not particularly limited as long as it can increase the viscosity of the composition and can be used in animal foods. Examples of thickeners include, but are not limited to, gum arabic, curdlan, processed starch, casein sodium, carrageenan, karaya gum, carob bean gum, xanthan gum, chitin, chitosan, guar gum, glucosamine, psyllium seed gum, gellan gum, tamarind gum, tara gum, pullulan, pectin, and methylcellulose and the like. In a preferred embodiment of the present invention, the thickener may be guar gum or processed starch.

When the thickener is contained in the first composition, the lower limit of the amount of the thickener is generally 0.01% or more, preferably 0.1% or more, 1% or more, or 3% or more, but is not limited thereto. The upper limit is generally 15% or less, preferably 10% or less, 9% or less, or 8% or less, but is not limited thereto. In one embodiment, the amount of the thickener contained in the first composition is generally 0.01 to 15%, preferably 0.1 to 10%, 1 to 9%, or 3 to 8%, but is not limited thereto.

In one embodiment, the first composition can be made into a paste by the thickener. By making the first composition into a paste, the handling of the pharmaceutical or food composition of the present invention becomes easy and convenient, which may be preferred.

The first composition may further contain other ingredients other than the fish meat or livestock meat component and cystine (and cystine alternative). Examples of other ingredients include, but are not limited to, pH adjuster, antioxidant, preservative, sweetener, flavor, seasoning, and nutrient fortifier. Specific examples of other ingredients include, but are not limited to, extracts (seafood extracts such as extract of tuna, extract of dried bonito flakes and the like, livestock meat extract such as chicken extract and the like, yeast extract and green tea extract, etc.), vitamins (vitamins A, B, C and E, etc.), dye (red yeast dye and carotenoid dye, caramel dye, etc.), saccharides other than reducing sugar, protein hydrolysate, vegetable fats and oils, minerals, seasoning (amino acid, etc.), and fungi(lactobacillus etc.) and the like. When the above-mentioned ingredients are contained in the first composition, there is no limit to the amount of the ingredients, and the amount may be appropriately set according to the property of the intended product.

In the present specification, "cystine alternative" refers to a substance that is permitted to be added to animal foods and a compound that has a thiol group in its chemical structure. Compounds having a thiol group in their chemical structure include, but are not limited to, 1-propanethiol, 2-methyl-1-propanethiol, cyclopentanethiol, 2-hydroxybenzenethiol, dihydrolipoic acid, L-cysteine, reduced glutathione, coenzyme A, and γ-glutamylcysteine.

The form of the first composition may be any of solid, semi-solid, paste, and liquid. In one embodiment, the first composition may be semi-solid, paste, or liquid. The water content of the first composition is generally 40 to 95%, and may be preferably 50 to 95%, 55 to 93%, or 60 to 90%, but is not limited to these. The "water content" of the first composition means the water content (also called the moisture content) contained in the first composition. The water content of the first composition can be calculated by the following formula. [Water content (wt%) of the first composition] = (weight of water contained in all components constituting the first composition (fish meat or livestock meat component, etc.)) x 100/(total weight of the first composition)

The water content may be measured by a method known per se. For example, the method described in the "Feed Analysis Standards (Notice 19 Shoan, No. 14729, by the Director-General of Food Safety and Consumer Affairs Bureau, Ministry of Agriculture, Forestry and Fisheries of Japan dated Apr. 1, 2008))" provided by the Food and Agricultural Materials Inspection Center can be mentioned, but the method is not limited thereto.

The first composition does not contain cystine (and cystine alternatives). In the present specification, the "does not contain cystine (and cystine alternatives)" encompasses not only an embodiment in which the first composition does not contain cystine (and cystine alternatives) at all, but also an embodiment in which the first composition contains cystine in an amount that generates a trace amount of hydrogen sulfide that does not cause health damage to animals.

### (Second composition)

The second composition contains cystine, but does not contain reducing sugar or phosphorus.

The cystine contained in the second composition is not particularly limited, so long as it is permitted to be added to pharmaceutical products or foods for animals, and may be prepared by a method known per se or commercially available. In one embodiment, the cystine may be L-cystine.

The lower limit of the amount of cystine in the second composition is generally 0.05% or more, preferably 0.1% or more, 0.5% or more, 1% or more, 1.5% or more, 2% or more, or 2.5% or more, but is not limited to these, based on the second composition. The upper limit is generally 10% or less, preferably 9% or less, 8% or less, 7% or less, 6% or less, 5% or less, or 4% or less, but is not limited to these. In one embodiment, the amount of cystine contained in the second composition is generally 0.05-10%, preferably 0.1-9%, 0.5-8%, 1-7%, 1.5-6%, 2-5% or 2.5-4%, but is not limited to these.

In another embodiment, the amount of cystine contained in the second composition is generally 1% or more, preferably 10% or more, 20% or more, 30% or more, 40% or more, 50% or more, 60% or more, 70% or more, 80% or more, or 90% or more, but is not limited to these. The upper limit is generally 100% or less, preferably 99% or less, 98% or less, 97% or less, 96% or less, 95% or less, 94% or less, 93% or less, 92% or less, or 91% or less, but is not limited to these. In one embodiment, the amount of cystine contained in the second composition is generally 1 to 100%, preferably 10 to 100%, 20 to 100%, 30 to 100%, 40 to 100%, 50 to 100%, 60 to 100%, 70 to 100%, 80 to 100%, or 90 to 100%, but is not limited to these.

The reducing sugar and phosphorus are the same as those described in the first composition. They are not contained in the second composition. In the present specification, the " does not contain reducing sugar and phosphorus" encompasses not only an embodiment in which the second composition does not contain reducing sugar and phosphorus at all, but also an embodiment in which the second composition contains reducing sugar and phosphorus in amounts that generate a trace amount of hydrogen sulfide that does not cause health damage to animals.

The second composition may contain other components in addition to cystine (and cystine alternatives), reducing sugar, and phosphorus. Examples of other components include, but are not limited to, thickeners, pH adjusters, antioxidants, and preservatives. In one embodiment, the second composition further includes a thickener. The thickener is the same as that described in the first composition. The thickener used in the first composition and the thickener used in the second composition may be the same or different.

When a thickener is contained in the second composition, the lower limit of the amount of the thickener is generally 0.01% or more, preferably 0.1% or more, 1% or more, or 3% or more, but is not limited to these. The upper limit is generally 15% or less, preferably 10% or less, 9% or less, or 8% or less, but is not limited to these. In one embodiment, the amount of the thickener contained in the second composition is generally 0.01 to 15%, preferably 0.1 to 10%, 1 to 9%, or 3 to 8%, but is not limited to these. For example, by forming the second composition into a paste, the handling of the pharmaceutical or food composition of the present invention becomes easier, which may be preferred.

In one embodiment, the pH of the second composition may be generally 2 or more, preferably 2.5 or more, 3 or more, or 3.5 or more, at room temperature (15 to 25°C). In one embodiment, the pH of the second composition may be generally 9 or less, preferably 8.5 or less, 8.0 or less, or 7.5 or less, at room temperature. In another embodiment, the pH of the second composition may be generally 2 to 9, preferably 2.5 to 8.5, 3 to 8, or 3.5 to 7.5, at room temperature. In another embodiment, the pH of the second composition may be generally 2 to 7, preferably 2.5 to 6.5, 2 to 6, or 2 to 5.5, at room temperature. In yet another embodiment, the pH of the second composition may be generally 3 to 9, preferably 4 to 8.5, 5 to 8, or 6 to 7.5, at room temperature.

### (High-temperature sterilization)

In a preferred embodiment, the first composition and the second composition may be sterilized at high temperature. High-temperature sterilization may be performed using equipment and conditions that are generally used in the production of pharmaceutical or food for animals, and are not particularly limited. Examples of the conditions for high-temperature sterilization include a sterilization temperature that is generally 100°C or higher, preferably 105°C or higher, 110°C or higher, 115°C or higher, or 120°C or higher, as a lower limit, and generally 150°C or lower, preferably 140°C or lower, 130°C or lower, or 125°C or lower, as an upper limit. In one embodiment, the sterilization temperature is generally 100 to 150°C, preferably 100 to 140°C, or 100 to 130°C. The heating time may be appropriately set in consideration of the sterilization temperature, and the lower limit may be generally 1 min or more, preferably 3 min or more, 5 min or more, 15 min or more, 30 min or more, or 45 min or more, and the upper limit may be generally 180 min or less, preferably 150 min or less, 120 min or less, 100 min or less, 90 min or less, or 60 min or less. In one embodiment, the sterilization time may be generally 1 to 180 min, preferably 3 to 150 min, 5 to 120 min, 15 to 100 min, 30 to 90 min, or 45 to 60 min.

In one embodiment, the high-temperature sterilization of the present invention may be retort sterilization (pressureheat sterilization). The retort sterilization conditions may be any known conditions as long as sterilization is possible.

### (Animal)

The animals to which the pharmaceutical or food composition of the present invention can be applied are not particularly limited as long as they have AIM, but mammals are preferred. Mammals include mammals classified into Hominidae, Canidae and Felidae. In particular, Felidae animals, as described above, have very strong binding between AIM and IgM pentamer, and free AIM is unlikely to form in the blood, making them suitable as subjects for the pharmaceutical or food composition of the present invention.

Animals classified in the Canidae include, but are not limited to, Urocyon cinereoargenteus, Urocyon littoralis, Otocyon megalotis, Nyctereutes procyonoides, Vulpes cana, Vulpes zerda, Vulpes chama, Vulpes lagopus, Vulpes macrotis, Vulpes corsac, Vulpes ferrilata, Vulpes rueppelli, Vulpes vulpes, Vulpes bengalensis, Vulpes pallida, Vulpes velox, Chrysocyon brachyurus, Speothos venaticus, Atelocynus microtis, Cerdocyon thous, Lycalopex vetulus, Lycalopex fulvipes, Lycalopex culpaeus, Lycalopex sechurae, Lycalopex griseus, Lycalopex gymnocercus, Lycaon pictus, Cuon alpinus, Canis adustus, Canis mesomelas, Canis aureus, Canis simensis, Canis anthus, Canis latrans, Canis rufus, Canis lycaon, Canis lupus, Canis lupus arctos, Canis lupus dingo, Canis lupus familiaris, and subspecies thereof. In a preferred embodiment of the present invention, the animal classified as Canidae is Canis lupus familiaris.

Furthermore, animals classified in the Felidae include, but are not limited to, Panthera leo, Panthera pardus, Panthera tigris, Panthera uncia, Panthera onca, Neofelis nebulosa, Neofelis diardi, Puma concolor, Acinonyx jubatus, Herpailurus yagouaroundi, Caracal aurata, Caracal caracal, Leptailurus serval, Catopuma badia, Catopuma temminckii, Pardofelis marmorata, Leopardus colocola, Leopardus geoffroyi, Leopardus guigna, Leopardus guttulus, Leopardus jacobita, Leopardus pardalis, Leopardus tigrinus, Leopardus wiedii, Lynx canadensis, Lynx lynx, Lynx pardinus, Lynx rufus, Prionailurus bengalensis, Prionailurus javanensis, Prionailurus planiceps, Prionailurus rubiginosus, Prionailurus viverrinus, Otocolobus manul, Felis bieti, Felis catus, Felis chaus, Felis lybica, Felis margarita, Felis nigripes, Felis silvestris, and subspecies thereof. In a preferred embodiment of the present invention, the animal classified as Felidae is Felis catus.

### (Container)

In the pharmaceutical or food composition of the present invention, the first composition and the second composition are filled in separate containers. There is no particular limitation on the container used in the present invention, so long as the container can be sealed after the composition is filled.

As described above, in one embodiment of the present invention, the first composition and/or the second composition are sterilized at high temperature. The high temperature sterilization may be performed on the container after the composition is filled and sealed. In such a case, it is preferable that the container is heat-resistant. Examples of heat-resistant container materials include, but are not limited to, polypropylene, polyvinylidene chloride, polycarbonate, and polyethylene terephthalate.

In another embodiment, the container filled with the first composition and the container filled with the second composition may be integrated by partially connecting the two containers (hereinafter, sometimes referred to as an "integrated container"). A specific example of an integrated container is, for example, PAKITTE (registered trademark) (produced by DISPEN PAK JAPAN), but is not limited to this. An example of an integrated container that can be used in the present invention is shown in Fig. 1. (a) shows a schematic diagram of the container as viewed from above. The dotted line indicates the part where the container can be folded. (b) shows a schematic diagram of the container as viewed from below. The parts filled with the first composition and the second composition are indicated by hatching. In the present invention, the integrated container may have a completely separated portion filled with the first composition and a portion filled with the second composition, or may be openly connected between a portion filled with the first composition and a portion filled with the second composition, as long as the first composition and the second composition are in a state where they are not substantially mixed together (for example, the first composition and the second composition are in contact with each other in a part, but do not mix because both compositions have a certain degree of viscosity) (schematic diagrams are shown in (c) and (d), respectively. Note that (c) and (d) are X-Y cross-sectional views of (a), and the portions filled with the first composition and the second composition are shown by hatching). Note that in Fig. 1, the two filling portions filled with the compositions are shown to be the same size, but the two filling portions may be different sizes. Also, the two filling portions may be configured to be symmetrical or asymmetrical.

In one embodiment, the integrated container used in the present invention may have a configuration as shown in Fig. 2. In Fig. 2, the portions filled with the first composition and the second composition are shown by hatching. In (a), the container portion filled with the first composition and the container portion filled with the second composition are connected at one end, and the connected portion may be configured to be separable. Fig. 2(b) shows an embodiment in which the container portion filled with the first composition and the container portion filled with the second composition are partially separated.

In another embodiment, the container filled with the first composition and the container filled with the second composition may be completely separated, as shown in Fig. 3(a). In Fig. 3, the portions filled with the first composition and the second composition are also shown by hatching. During the production step, a container filled with the first composition and a container filled with the second composition may be separately produced as shown in (a), and finally these may be combined and packaged in a packaging bag to prepare the pharmaceutical or food composition of the present invention.

Since the pharmaceutical or food composition of the present invention has the effect of increasing the amount of free AIM in the blood of animals, it can be suitably used for the treatment or prevention of various diseases, but when given to Felidae in particular, it can be a pharmaceutical or food composition for the treatment and/or prevention of renal diseases (acute renal failure, chronic renal failure).

The amount of the pharmaceutical or food composition of the present invention to be fed to Felidae animals can be appropriately determined taking into consideration the health condition, weight, age, and the like of the animal.

In addition, the pharmaceutical or food composition of the present invention may be used in combination with existing agents for treating or preventing renal diseases that are used for Felidae animals.

### 2. Method for producing pharmaceutical or food composition for animals

The present invention also provides a method for producing a pharmaceutical or food composition for animals, including a first composition and a second composition in combination, the method including a step of filling a first container with a first composition containing a fish meat or livestock meat component but not containing cystine, and a step of filling a second container with a second composition containing cystine but not containing reducing sugar or phosphorus (hereinafter, this method is sometimes to be referred to as the "production method of the present invention").

The first composition, the second composition, the animal, the container, and the like in the production method of the present invention are the same as those described in the pharmaceutical or food composition of the present invention.

In one embodiment, the production method of the present invention further comprises the step of subjecting the first container filled with the first composition and the second container filled with the second composition to high-temperature sterilization.

The conditions for high-temperature sterilization are also the same as those described in the pharmaceutical or food composition of the present invention.

The present invention is described in more detail in the following examples, but the present invention is not limited to these examples.

### [Examples]

### [Example 1] Examination of hydrogen sulfide generation conditions 1

The following test was conducted to confirm which of the many ingredients generally contained in pet food contributes to the generation of hydrogen sulfide derived from cystine.

In consideration of the results of a preliminary experiment (not disclosed in the present specification), whether three factors, (1) reducing sugar, (2) phosphorus, and (3) NaCl, affect the generation of hydrogen sulfide derived from cystine was examined. Glucose ("water-containing crystal glucose", manufactured by SAN-EI SUCROCHEMICAL CO., LTD.) was used as the reducing sugar, "potassium dihydrogen phosphate" (manufactured by Yoneyama Chemical Industry Co., Ltd.) was used as the phosphorus, and "Nakuru M" (manufactured by Naikai Salt Industry Co., Ltd.) was used as the NaCl.

As shown in Tables 1 to 3 below, samples were prepared containing L-cystine (manufactured by NIPPON RIKA CO., LTD.), polysaccharide thickener ("ORNO G1", ORGANO FOODTECH CORPORATION), water (purified water), and any of (1) to (3). Note that descriptions such as "0.5-fold amount" refer to the molar ratio of reducing sugar, phosphorus, or NaCl to L-cystine. Note that "%" in the Tables refers to "% by weight".

**[Table 1]**

| | negative control | 0.5-fold amount reducing sugar | 1-fold amount reducing sugar | 1.5-fold amount reducing sugar | 2-fold amount reducing sugar |
|---|---|---|---|---|---|
| reducing sugar | 0 | 1.34% | 2.68% | 4.00% | 5.36% |
| L-cystine | 3.57% | | | | |
| polysaccharide thickener | 1.40% | | | | |
| water | 95.03% | 93.69% | 92.35% | 91.01% | 89.67% |
| total | 100% | | | | |

**[Table 2]**

| sample name | negative control | 0.1-fold amount phosphorus | 0.2-fold amount phosphorus | 0.3-fold amount phosphorus | 0.4-fold amount phosphorus |
|---|---|---|---|---|---|
| potassium dihydrogen phosphate | 0 | 0.091% | 0% | 0.273% | 0.360% |
| disodium hydrogen phosphate | 0 | 0.116% | 0.232% | 0.348% | 0.460% |
| L-cystine | 3.57% | | | | |
| polysaccharide thickener | 1.40% | | | | |
| water | 95.03% | 94.82% | 94.80% | 94.41% | 94.20% |
| total | 100% | | | | |

**[Table 3]**

| sample name | negative control | 1-fold amount NaCl | 2-fold amount NaCl | 3-fold amount NaCl | 4-fold amount NaCl |
|---|---|---|---|---|---|
| NaCl | 0 | 0.87% | 1.74% | 2.61% | 3.47% |
| L-cystine | 3.57% | | | | |
| polysaccharide thickener | 1.40% | | | | |
| water | 95.03% | 94.16% | 93.29% | 92.42% | 91.55% |
| total | 100% | | | | |

A pouch packaging material (90 mm x 140 mm) was filled with 45.0 g of a sample having the composition shown in each Table above, and sterilized by heating at 120°C for 25 min using a retort sterilizer (manufactured by HISAKA WORKS, LTD.). The sample was left at 25°C for 24 hr after sterilization. The sample was then opened, and 40.0 g of the sample was weighed out and placed in a 100 mL vial, sealed with parafilm, and left at 25°C for 1 hr in a thermostatic chamber. The generation of hydrogen sulfide was measured using a GASTEC detector tube (GV-110s, manufactured by GASTEC CORPORATION) before sterilization and at 1 hr after opening. Measurements were performed with n=3. The results of reducing sugar (glucose) and phosphorus are shown in Tables 4 and 5 below.

**[Table 4]**

| reducing sugar (glucose) | | |
|---|---|---|
| molar ratio (fold) | average (ppm) | |
| | before sterilization | 1 hr after opening |
| 0 | 0 | 8.5 |
| 0.5 | 0 | 41.7 |
| 1 | 0 | 52.8 |
| 1.5 | 0 | 84.2 |
| 2 | 0 | 82.4 |

**[Table 5]**

| phosphorus | | |
|---|---|---|
| molar ratio (fold) | average (ppm) | |
| | before sterilization | 1 hr after opening |
| 0 | 0 | 8.3 |
| 0.1 | 0 | 139.8 |
| 0.2 | 0 | 300 |
| 0.3 | 0 | 406.7 |
| 0.4 | 0 | 500 |

As shown in Tables 4 and 5, reducing sugar (glucose) and phosphorus were shown to affect the generation of hydrogen sulfide derived from cystine. It was also shown that the amount of hydrogen sulfide generated tends to increase as the amount of reducing sugar or phosphorus in the sample increases. On the other hand, for NaCl, hydrogen sulfide was not substantially detected in any of the samples, both before sterilization and at 1 hr after opening.

### [Example 2] Examination of hydrogen sulfide generation conditions 2

Next, whether pH conditions affect the generation of hydrogen sulfide was examined. The samples shown in Table 6 below were prepared.

**[Table 6]**

| sample name | pH 3.5 | pH 7.0 | pH 9.5 |
|---|---|---|---|
| L-cystine | 3.57% | | |
| thickener | 1.40% | | |
| citric anhydride | 0.32% | 0.0052% | 0% |
| trisodium citrate hydrate | 0.10% | 0.4148% | 0% |
| sodium carbonate (0.1 mol/L) | 0% | 0% | 1.06% |
| purified water | 94.61% | 94.61% | 93.97% |
| total | 100% | | |

A pouch packaging material (90 mm x 140 mm) was filled with 45.0 g of samples having the compositions shown in the above tables, and sterilized by heating at 120°C for 25 min using a retort sterilizer (manufactured by HISAKA WORKS, LTD.). After sterilization, the sample was left at rest at 25°C for 24 hr. The sample was then opened, and 40.0 g was weighed out and placed in a 100 mL vial, sealed with parafilm, and left at 25°C for 1 hr in a thermostatic chamber. The generation of hydrogen sulfide was measured using a GASTEC detector tube GV-110s (manufactured by GASTEC CORPORATION) before sterilization and at 1 hr after opening. Measurements were performed with n=3.

The results are shown in Table 7 below.

**[Table 7]**

| pH | average (ppm) | |
|---|---|---|
| | before sterilization | 1 hr after opening |
| 3.5 | 0 | 34 |
| 7.0 | 0 | 8.5 |
| 9.5 | 0 | 198.1 |

As shown in Table 7, almost no hydrogen sulfide was generated at pH 3.5 and 7.0. On the other hand, hydrogen sulfide was generated at pH 9.5.

### [Industrial Applicability]

The pharmaceutical or food composition of the present invention is particularly preferable for use in the treatment and/or prevention of renal diseases in Felidae. Therefore, the pharmaceutical or food composition of the present invention and the production method thereof are extremely useful in the field of medical care for Felidae animals.

This application is based on a patent application No. 2022-013891 filed in Japan (filing date: February 1, 2022), the contents of which are incorporated in full herein.

## Claims

1. A pharmaceutical or food composition for animals, comprising a first composition and a second composition in combination,
wherein the first composition comprises a fish meat or livestock meat component but does not comprise cystine,
the second composition comprises cystine but does not comprise reducing sugar or phosphorus, and
the first composition and the second composition are filled in separate containers.

2. The composition according to claim 1, wherein the first composition further comprises either or both of reducing sugar and phosphorus.

3. The composition according to claim 1 or 2, wherein the second composition has a pH of 3 to 9.

4. The composition according to claim 1 or 2, wherein the first composition and the second composition are sterilized at a high temperature.

5. The composition according to claim 4, wherein the high-temperature sterilization is performed under conditions of 100 to 150°C and 1 to 180 minutes.

6. The composition according to claim 1 or 2, wherein the animal is Canidae or Felidae.

7. The composition according to claim 6, wherein Felidae is Felis catus.

8. The composition according to claim 1 or 2, wherein a container filled with the first composition and a container filled with the second composition are connected.

9. A method for producing a pharmaceutical or food composition for animals, comprising a first composition and a second composition in combination, the method comprising
a step of filling a first container with a first composition comprising a fish meat or livestock meat component but not comprising cystine, and
a step of filling a second container with a second composition comprising cystine but not comprising reducing sugar or phosphorus.

10. The production method according to claim 9, wherein the first composition further comprises either or both of reducing sugar and phosphorus.

11. The production method according to claim 9 or 10, wherein the second composition has a pH of 3 to 9.

12. The production method according to claim 9 or 10, further comprising a step of subjecting the first container filled with the first composition and the second container filled with the second composition to high-temperature sterilization.

13. The production method according to claim 12, wherein the high-temperature sterilization is performed under conditions of 100 to 150°C and 1 to 180 minutes.

14. The production method according to claim 9 or 10, wherein the animal is Canidae or Felidae.

15. The production method according to claim 14, wherein Felidae is Felis catus.

16. The production method according to claim 9 or 10, wherein the container filled with the first composition and the container filled with the second composition are connected.
